# EUROPEAN PATENT APPLICATION

(11) **EP 3 536 305 A1**
(43) Date of publication of application: **11.09.2019**
(21) Application number: 18160498.4
(22) Date of filing: 07.03.2018
(51) Int. Cl.: A61K 6/10, A61K 6/087

(54) **DENTAL IMPRESSION MATERIAL**

(71) Applicant: Dentsply DeTrey GmbH, 78467 Konstanz (DE)
(72) Inventor: KLEE, Joachim E., 78315 Radolfzell (DE); SZILLAT, Florian, 78462 Konstanz (DE); WÖRNER, Melissa, 78462 Konstanz (DE); LALEVÉE, Jacques, 68200 Mulhouse (FR); KIRSCHNER, Julie, 67650 Dambach-la-Ville (FR); MORLET-SAVARY, Fabrice, 68120 Pfastatt (FR); DIETLIN, Céline, 68100 Mulhouse (FR); WEBER, Christoph, 78464 Konstanz (DE)
(74) Representative: Schiener, Jens

(57) **Abstract**

A curable dental impression material comprising:
(a) one or more siloxane compounds having two or more epoxide groups;
(b) a photo initiator for initiating cationic polymerization of epoxide groups of the one or more siloxane compounds; and
(c) a filler.

## Description

### Field of the Invention

The present invention relates to a curable dental impression material. Moreover, the present invention relates to a method for preparing a dental impression. Finally, the present invention relates to a use of the curable dental impression material of the present invention for the preparation of a dental impression.

### Background of the Invention

Curable dental impression materials containing polyorganosiloxane compositions are known. Dental impression materials are commonly available as reactive multicomponent materials provided in packages including two compartments or two separate containers that keep the components isolated from each other during storage. Once the components are mixed, a chemical reaction is initiated that turns the mixed composition into a hardened mass during the setting time. Moreover, the working time and the setting time of conventional dental impression materials are limited and depend on the rate of the curing reaction. Therefore, storage stability of a dental impression material depends on the separation of reactive components and necessitates mixing prior to use which needs to be done chairside immediately prior to use so that the dental impression may be completed during the working time of usually only a few minutes.

In view of the stability-reactivity problems inherent in conventional dental impression materials, devices have been developed for the automatic mixing and dispensing of dental impression materials to provide high precision regarding the homogeneity of the mixture, and the ratio of the two components to be mixed. Accordingly, the components of the dental impression material are simultaneously supplied from separate material chambers to a mixer during application of the dental impression material, which mixes and then dispenses a mixed paste. The paste may be supplied from the mixer directly onto a dental impression tray for immediate placement in a patient's mouth.

Once the material components have contact with each other in the mixing chamber, the mixture of the material in the mixing chamber can only be stored for a short time because the mixed material will soon set inside the mixing chamber unless dispensed and used. Therefore, the dental practitioner may have to remove and replace mixers several times each day.

Dental impression materials may be silicone impression material curable in an addition or condensation reaction whereby addition silicones are most popular. The conventional addition silicone impression materials provide good detail reproduction, excellent dimensional stability, little shrinkage on set, addition silicones are inherently hydrophobic and as such require moisture control for optimal use. Nevertheless, addition silicones have only a poor tear resistance.

Therefore, curable dental impression materials must meet the following requirements.
1. The materials should be stable over at least two years.
2. The materials should be highly tolerant to moisture.
3. The materials should be one-part visible light-polymerizable compositions to be easy to apply.
4. The curable dental impression material should be hydrophilic, because otherwise the hydrophobicity of the impression material can result in a loss of surface detail at critical surfaces of the dentition.
5. Moreover, the curing time of the dental impression material should be short. A curing time of about 20-45 seconds reduces the risk of a positional shift of the lower jaw from the position first adopted by the patient.
6. For photoinitiated curing of the material the depth of the photopolymerization is very important to guarantee an entirely cured dental impression material.
7. Furthermore, a high conversion of the polymerizable groups should be achieved after the curing process to avoid leaching of unreacted monomers out of the polymerized dental composition and to achieve optimal mechanical strength.
8. After the curing process of the dental impression material the impression needs to be easily removable from the dentition without destroying sensitive areas of the cured impression material. Therefore, the cured dental impression material must be non-adherent to the tooth.
9. The curable dental impression material must provide a high fidelity of structural reproduction for fidelity of oral prosthetic fit.
10. The surface of the cured dental impression material must be free from irregularities, blemishes, pits and other imperfections.
11. Furthermore, the material needs a high retention of shape after the curing process, a high tear strength, final hardness, stable consistency and it should not be sensitive to temperature.
12. Additionally, the material should be transparent.

Dental impression material based on cross-linking polysiloxanes are known. For example, US-A-5849812 describes an addition-curing polyether dental impression material comprising (a) at least one polyether which has at least two optionally substituted vinyl and/or allyl end-groups, (b) an SiH component, (c) at least one platinum catalyst, (d) optional additives, and (e) organopolysiloxane with at least two alkenyl groups.

EP 1 465 579 B1 describes a ternary photoinitiator system for cationically polymerizable resins comprising of (a) an iodonium salt, (b) a visible light sensitizer and (c) an electron donor compound.

EP 0 789 721 B1 describes a visible-light curable epoxy system with enhanced depth of cure consisting of (a) a cationically polymerizable epoxy resin, (b) a hydroxyl-containing material, (c) an aryliodonium salt and (d) a visible-light sensitizer.

### Summary of the Invention

However, the aforementioned patent documents do not disclose photoinitiated cationically polymerizable dental impression materials, which meet the strict requirements for such materials mentioned above.

It is the problem of the present invention to provide a curable dental impression material which has high storage stability over several months up to one year, which is sufficiently hydrophilic, which is highly tolerant to moisture, provides a fidelity of structural reproduction, and which may be provided as a single composition which does not need mixing prior to use.

Moreover, it is the problem of the present invention to provide a curable dental impression material which composition provides
- good polymerization efficiency including a high conversion and a good curing rate which may be adapted to provide a suitable working time of the composition,
- high depth of cure, and
- high retention of shape, high tear strength, final hardness, stable consistency and temperature stability.

It is a further problem of the present invention to provide a curable dental impression material which is easily removable from the dentition after the curing process and the surface of the cured dental impression material must be free from irregularities, blemishes, pits and other imperfections.

Furthermore, it is a problem of the present invention to provide a curable dental impression material which is a one-part visible light-polymerizable composition to be easy to apply.

It is a further problem of the present invention to provide a method for preparing a dental impression which does not require strict moisture control, wherein working and setting times may be determined by the dental practitioner, whereby the cured dental impression material has excellent tear resistance and provides at the same time good detail reproduction, excellent dimensional stability, and reduced shrinkage on set.

Finally, it is the problem of the present invention to provide a use of the curable dental impression material of the invention for the preparation of a dental impression.

Accordingly, the present invention provides (1) a curable dental impression material comprising:
(a) one or more siloxane compounds having two or more epoxide groups;
(b) a photo initiator for initiating cationic polymerization of epoxide groups of the one or more siloxane compounds; and
(c) a filler.

Furthermore, the present invention provides a method for preparing a dental impression, which method comprises
(a) providing a curable dental impression material as defined by (1);
(b) taking an impression of a dental structure;
(c) curing the dental impression material with light having a wavelength in the range of 200 to 800 nm.

Finally, the present invention provides a use of the curable dental impression material as defined by (1), for the preparation of a dental impression.

### Effects of the invention

The present invention is based on the recognition that a curable dental impression material according to the present invention comprising (a) one or more siloxane compounds having two or more epoxide groups, (b) a photo initiator for initiating cationic polymerization of epoxide groups of the one or more siloxane compounds and (c) a filler, provides a good polymerization efficiency, a short curing time of 20 seconds, a high depth of cure, a high storage stability.

### Brief description of the Figures

Fig. 1 shows photopolymerization profiles of epoxy functions for poly[dimethylsiloxane-*co*-(2-(3,4-epoxycyclohexyl)ethyl)methylsiloxane] (silcolease UV Poly 200 from bluestar silicones) upon SmartLite Focus Irradiation (300 mW/cm²; Under Air; Thickness = 1.4 mm) using a CQ/Ph3GeH/Bluesil 2074 (1/1.2/1.5% w/w) initiating system.
Fig. 2 shows depth of cure for the polymerization upon blue light of poly[dimethylsiloxane-*co-*(2-(3,4-epoxycyclohexyl)ethyl)methylsiloxane] (silcolease UV Poly 200 from bluestar silicones) upon SmartLite Focus Irradiation using different initiating systems.

### Detailed Description of Preferred Embodiments

The present invention provides a curable dental impression material.

### The siloxane compounds having two or more epoxide groups

The curable dental impression material of the present invention comprises one or more siloxane compounds having two or more epoxide groups.

Preferably, the siloxane compound is a compound of the following formula (I):

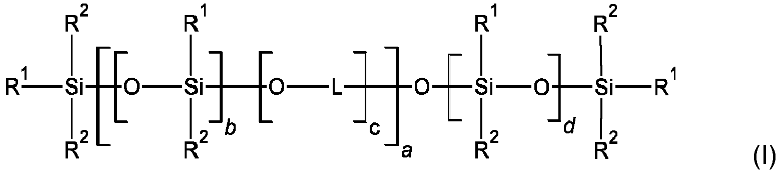

At least two epoxy groups are present in the compound of formula (I).

In formula (I), R¹ which may be the same or different, independently preferably represents an epoxide-group containing organic residue, a C₁₋₄ alkyl group, a group of the formula [**-OSiR**'₂]ₙ**R'**, or two groups **R¹** or a group R¹ and R² form together a group [**-OSiR'**₂]ₙ**-**, wherein the **R'**, which may be the same or different, independently represent a C₁₋₄ alkyl group or an epoxide-group containing organic residue and n is an integer of from 1 to 20.

More preferably, R¹ represents an epoxide-group containing organic residue.

The epoxide-group containing organic residues are cycloaliphatic epoxide groups of the following formula (II):

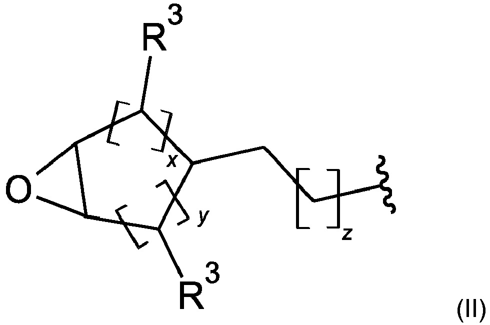

In formula (II), R³ which may be the same or different, independently preferably represent a hydrogen atom or a C₁₋₄ alkyl group, more preferably a hydrogen atom.

In formula (II), x preferably is 0 or an integer of from 1 to 4, more preferably it is 1.
In formula (II), y preferably is 0 or an integer of from 1 to 4, more preferably y is 2.

In formula (II), z preferably is an integer of from 1 to 3.

In formula (II), (x+y) preferably is 2 to 8, more preferably it is 3.

In formula (I), R² which may be the same or different, independently preferably is a C₁₋₄ alkyl group or a group of the formula [**-OSiR"**₂]ₘ**R"**, or two groups **R**² or a group R¹ and R² form together a group [**-OSiR"**₂]ₘ-, wherein the R", which may be the same or different, independently represent a C₁₋₄ alkyl group and m is an integer of from 1 to 20.

More preferably, R² is a C₁₋₄ alkyl group, most preferably R² is a methyl group.

In formula (I), the divalent linker group L preferably is a hydrocarbon group which may be aliphatic and/or aromatic, preferably aliphatic, and preferably has 1 to 20 carbon atoms. The aliphatic hydrocarbon group may be saturated or unsaturated.

The hydrocarbon group may be substituted with 1 to 6 C₁₋₄ alkyl groups. Specific examples of the alkyl groups are methyl, ethyl, n-propyl, i-propyl, n-butyl, i-butyl or tert.-butyl. The linker group L may contain 1 to 20 heteroatoms selected from oxygen, nitrogen and sulphur. The oxygen atoms, nitrogen atoms and sulphur atoms in the hydrocarbon group may be in the form of ether or thioether bonds, amine bonds, keto or sulfoxide groups, carboxylic acid or ester groups, amide groups, sulfonic acid or ester groups, hydroxyl groups and thiol or thioester groups.

In formula (I), a preferably represents 0 or an integer of from 1 to 20.

In formula (I), b preferably represents 0 or an integer of from 1 to 20.

In formula (I), c preferably represents 0 or an integer of from 1 to 20, more preferably c represents 0.

In formula (I), d preferably represents 0 or an integer of from 1 to 20.
provided that at least two epoxy groups are present in the compound of formula (I).

It is preferred that in formula (I), residues R¹ and R² are selected that the monomer has an average molecular weight of 200 to 10000, more preferably 500 to 5000 Da.

The monomer according to formula (I) is preferably selected in view of a good processibility and applicability of the curable dental impression material, in particular in terms of viscosity. Therefore, the dynamic viscosity (25 °C) of the compound of formula (I) is preferably in the range of from 0.1 to 1000 **Pa·s**, more preferably in the **range of from 1 to 100 Pa·s.**

Preferably, the curable dental impression material comprises 40 to 90 percent, more preferably 45 to 85 percent by weight based on the total weight of the dental impression material of siloxane compounds having two or more epoxide groups.

### The photo initiator

The curable dental impression material of the present invention comprises a photo initiator for initiating cationic polymerization of epoxide groups of the one or more siloxane compounds.

**The term "photo initiator system" refers to a system comprising at least** (a) one compound selected from diaryliodonium salts, triarylsulfonium salts, tetraarylphosphonium salts, or pyridinium salts, and (b) a photosensitizer.

**The term "photo sensitizer" refers to a molecule that produces a chemical change in** another molecule such as the photo initiator in a photochemical process. Preferably, the photo initiator comprises a sensitizer having an absorption maximum in the range of from 400 to 800 nm.

Suitable photo sensitizers may be selected from the group consisting of camphorquinone, benzil, 2,2'-3 3'- and 4,4'-dihydroxylbenzil, 2,3-butanedione, 2,3-pentanedione, 2,3-hexanedione, 3,4-hexanedione, 2,3-heptanedione, 3,4-heptanedione, 2,3-octanedione, 4,5-octanedionefuril, biacetyl, 1,2-cyclohexanedione, 1,2-naphthaquinone, and acenaphthaquinone. Camphorquinone is preferred.

Moreover, suitable Si- or Ge-acyl photo sensitizers may be selected from the (III):

X-R⁴ (III)

wherein
X is a group of the following formula (IV): 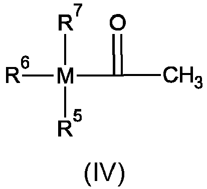 wherein
   M is Si or Ge;
   R⁵ represents a substituted or unsubstituted hydrocarbyl or hydrocarbylcarbonyl group;
   R⁶ represents a substituted or unsubstituted hydrocarbyl or hydrocarbylcarbonyl group;
   R⁷ represents a substituted or unsubstituted hydrocarbyl group; and
R⁴ i) has the same meaning as III, whereby the compound of formula (III) may be symmetrical or unsymmetrical; or
   ii) is a group of the following formula (V): 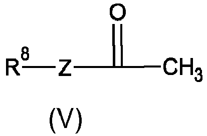 wherein
   Z represents a single bond, an oxygen atom or a group
      **NR', wherein R' represents a substituted or** unsubstituted hydrocarbyl group;
   R⁸ represents a substituted or unsubstituted hydrocarbyl group, a trihydrocarbylsilyl group, a mono(hydrocarbyl-carbonyl)dihydrocarbylsilyl group or a di(hydrocarbyl-carbonyl)monohydrocarbylsilyl group.

In connection with the Si- or Ge-acyl compound of formula (III), the term **"substituted" as used herein means that** R⁵, R⁶, R⁷, R⁸ and R' may be substituted by a substituent selected from the group consisting of halogen atoms, a nitro group, a cyano group, a hydroxy group, an amino group, C₁₋₆ alkyl groups, C₁₋₆ alkoxy groups and a -NR^{x}R^{y} group wherein R^{x} and R^{y} independently from each other represent a C₁₋₆ alkyl group. Here, illustrative of the halogen atoms can be fluorine, chlorine, bromine and iodine. The C₁₋₆ alkyl groups are, for example, methyl, ethyl, n-propyl, isopropyl and n-butyl. Illustrative of the C₁₋₆ alkoxy groups are, for example, methoxy, ethoxy and propoxy. The alkyl moieties in these substituents may be linear, branched or cyclic. Preferably, the substituent is selected from a chlorine atom, a nitro group, a C₁₋₄ alkoxy group and a -NR^{x}R^{y} group wherein R^{x} and R^{y} independently from each other represent a C₁₋₄ alkyl group.

If R⁵, R⁶ and R⁷ are substituted, then it is preferred that they are substituted with 1 to 3 substituents, more preferably with 1 substituent.

In the compound of formula (III), moieties R⁵, R⁶ and R⁷ may be defined as follows:
R⁵ and R⁶ independently from each other represent a substituted or unsubstituted hydrocarbyl or hydrocarbylcarbonyl group, and R⁷ represents a substituted or unsubstituted hydrocarbyl group.

The hydrocarbyl group may be an alkyl group, a cycloalkyl group, a cycloalkylalkyl group, an arylalkyl group or an aryl group.

An alkyl group may be straight-chain or branched C₁₋₂₀ alkyl group, typically a C₁₋₈ alkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a C₃₋₂₀ cycloalkyl group, typically a C₃₋₈ cycloalkyl group. Examples of the cycloalkyl group can include those having 3 to 6 carbon atoms, for example, cyclopropyl, cyclobutyl, cyclopentyl and cyclohexyl.

A cycloalkylalkyl group may have 4 to 20 carbon atoms and may include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl.

An arylalkyl group may be a C₇₋₂₀ arylalkyl group, typically a combination of a linear or branched alkyl group having 1 to 6 carbon atoms and an aryl group having 6 to 10 carbon atoms. Specific examples of an arylalkyl group are a benzyl group or a phenylethyl group.

An aryl group can include aryl groups having 6 to 10 carbon atoms. Examples of the aryl group are phenyl and naphtyl.

The hydrocarbylcarbonyl groups of R⁵ and R⁶ represent acyl groups (R_{org}-(C=O)-) in which the organic residue R_{org} is a hydrocarbyl residue as defined above.

Compound of formula (III) may contain one or two hydrocarbylcarbonyl groups, that is either one of R⁵ or R⁶ is a hydrocarbylcarbonyl group, or both R⁵ and R⁶ are hydrocarbylcarbonyl groups. Preferably, compound of formula (III) contains one hydrocarbylcarbonyl group.

Preferably, the hydrocarbylcarbonyl group is an arylcarbonyl group, more preferably a benzoyl group.

Preferably, R⁵ and R⁶ are independently selected from the group consisting of a straight chain or branched C₁₋₆ alkyl group, and a phenyl or benzoyl group which may optionally be substituted by one to three substitutents selected from halogen atoms, a nitro group, a C₁₋₄ alkoxy group and a -NR^{x}R^{y} group wherein R^{x} and R^{y} independently from each other represent a C₁₋₄ alkyl group, and R⁷ is a straight chain or branched C₁₋₆ alkyl group or a phenyl group.

Most preferably, R⁵ and R⁶ are independently selected from the group consisting of a straight chain or branched C₁₋₄ alkyl group, and a phenyl or benzoyl group which may optionally be substituted with one substituent selected from the group consisting of selected from a halogen atom, a nitro group, a C₁₋₄ alkoxy group and a -NR^{x}R^{y} group wherein R^{x} and R^{y} independently from each other represent a C₁₋₄ alkyl group, and R⁷ is a straight chain or branched C₁₋₄ alkyl group.

In the compound of formula (III), R⁴ may have the same meaning as X, whereby the compound of formula (III) may be symmetrical or unsymmetrical. Alternatively, R⁴ may represent a substituted or unsubstituted hydrocarbyl group, or a group of formula (V). Preferably, if R⁴ has the same meaning as X, then compound of formula (III) is unsymmetrical. If R⁴ represents a substituted or unsubstituted hydrocarbyl group, then the hydrocarbyl group has the same meaning as defined above for R⁵ and is independently selected therefrom.

In the group of formula (V) of compound of formula (III), R⁸ represents a substituted or unsubstituted hydrocarbyl group, a trihydrocarbylsilyl group, a mono(hydrocarbylcarbonyl)-dihydrocarbylsilyl group or a di(hydrocarbylcarbonyl)monohydrocarbylsilyl group.

If R⁸ of formula (V) is a trihydrocarbylsilylgroup, a mono(hydrocarbylcarbonyl)-dihydrocarbylsilyl group or a di(hydrocarbylcarbonyl)monohydrocarbylsilyl group, each of the hydrocarbyl and hydrocarbylcarbonyl groups has the same meaning as defined for R⁵, R⁶ and R⁷ and is independently selected therefrom.

In formula (V), R' has the same meaning as defined for R⁷ and is independently selected therefrom.

For example, compounds of formula (III) wherein R⁴ has the same meaning as X and which are symmetrical may be have the following structural formulae:

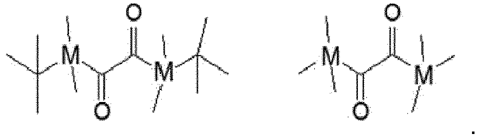

For example, compounds of formula (III) wherein R⁴ represents a group of formula (V) wherein Z is a bond, an oxygen atom or a NR' group, and R⁸ represents a substituted or unsubstituted hydrocarbyl group may have the following structural formulae:

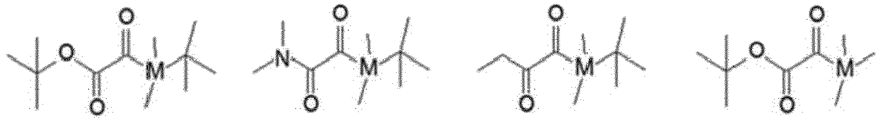

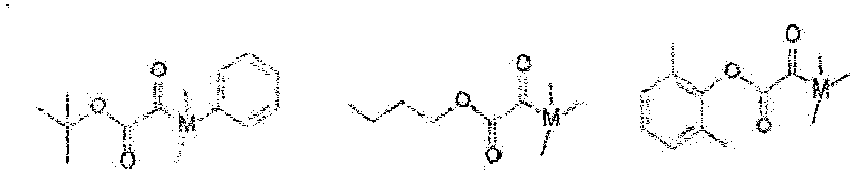

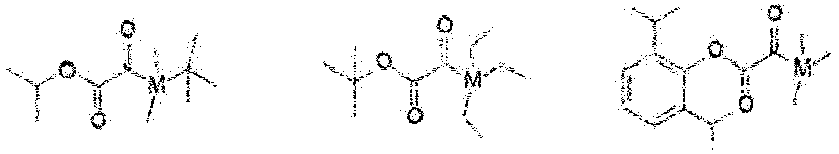

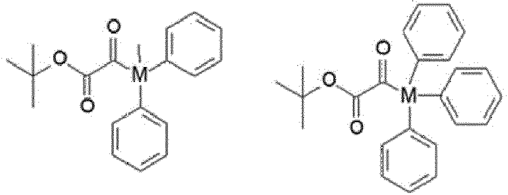

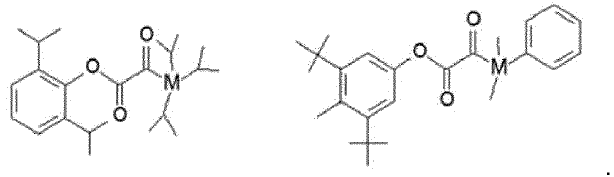

For example, compounds of formula (III) wherein R⁴ represents a group of formula (V) wherein R⁸ represents a trihydrocarbylsilyl group have the following structural formulae:

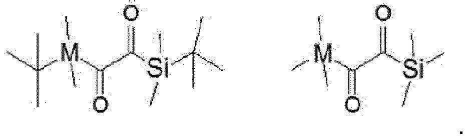

Preferably, compound of formula (III) is selected from the group consisting of: 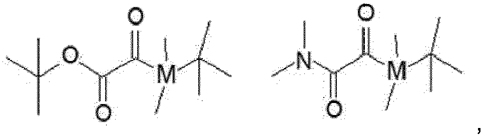 wherein compounds of formula (III) with M = Si are particularly preferred.

More preferably, compound of formula (III) has the following structural formula: 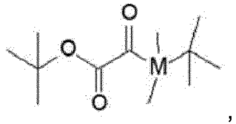 wherein it is particularly preferred that M = Si. That is, tert-butyl (tertbutyldimethylsilyl)-glyoxylate) (DKSi) is particularly preferred.

Preferably, the photo initiator system comprises at least (a) one compound selected from diaryliodonium salts including DPI, triarylsulfonium salts, tetraarylphosphonium salts, or pyridinium salts, and (b) camphor quinone (CQ) or DKSi, optionally in combination with a metal hydride derivative such as Ph₃GeH, an amine derivative or a sulfinate derivative such p-toluyl sulfinate. Alternatively, CQ/Ph₃GeH may be used.

Most preferably, DPI/CQ/amine; DPI/CQ/sulfinate; CQ/Ph₃GeH optionally DPI and DPI/DKSi is used.

Preferably, the diaryl iodonium including DPI, triaryl sulfonium, and tetraaryl phosphonium salts are selected from the following group:
(1) an diaryliodonium compound of the following formula (VI):

   R⁹-I⁺-R¹⁰ A⁻ (VI)

   wherein
   R⁹ and R¹⁰
   which are independent from each other, represent an optionally substituted aryl group, and
   A⁻ is an anion; and/or
(2) a triarylsulfonium compound of the following formula (VII):

   R¹¹R¹²R¹³S⁺ A⁻ (VII)

   wherein
   R¹¹, R¹² and R¹³
   which are independent from each other, represent an optionally substituted aryl group or wherein any two of R¹¹, R¹² and R¹³ form a cyclic aromatic structure together with the sulfur atom to which they are bound, and
   A⁻ is an anion; and/or
(3) a tetraarylphosphonium compound of the following formula (VIII):

   R¹⁴R¹⁵R¹⁶R¹⁷P⁺ A⁻ (VIII)

   wherein
   R¹⁴, R¹⁵, R¹⁶ and R¹⁷
   which are independent from each other, represent an optionally substituted aryl group or wherein any two of R¹⁴, R¹⁵, R¹⁶ and R¹⁷ form a cyclic aromatic structure together with the phosphor atom to which they are bound, and
   A⁻ is an anion.

Preferably, R⁹ and R¹⁰ of the diaryl iodonium compounds of formula (VI) which may be the same or different, R¹¹, R¹² and R¹³ of the triaryl sulfonium salt of formula (VII) which may be the same or different, R¹⁴, R¹⁵, R¹⁶ and R¹⁷ of the tetraaryl phosphonium salt of formula (VIII) which may be the same or different, independently represent optionally substituted aromatic group. An aromatic group may be a phenyl group. The phenyl group may be substituted by one or more straight chain or branched alkyl groups having 1 to 6 carbon atoms, straight chain or branched alkoxy groups having 1 to 6 carbon atoms, aromatic groups such as aryl groups or aryloxy groups, alicyclic groups having 3 to 6 carbon atoms, halogen atoms, hydroxyl groups, or amino groups.

Examples of useful diaryl iodonium salt include (4-methylphenyl)[4-(2-methylpropyl) phenyl] iodonium hexafluoroantimonate, include (4-methylphenyl)[4-(2-methylpropyl) phenyl] iodonium tetrafluoroborate, diphenyliodonium tetrafluoroborate, di(4-methylphenyl)iodonium tetrafluoroborate, phenyl-4-methylphenyliodonium tetrafluoroborate, di(4-heptylphenyl)iodonium tetrafluoroborate, di(3-nitrophenyl)iodonium hexafluorophosphate, di(4-chlorophenyl)iodonium hexafluorophosphate, di(naphthyl)iodonium tetrafluoroborate, di(4-trifluoromethylphenyl)iodonium tetrafluoroborate, diphenyliodonium hexafluorophosphate, di(4-methylphenyl)iodonium hexafluorophosphate; diphenyliodonium hexafluoroarsenate, di(4-phenoxyphenyl)iodonium tetrafluoroborat, phenyl-2-thienyliodonium hexafluorophosphate, 3,5-dimethylpyrazolyl-4-phenyliodonium hexafluorophosphate, diphenyliodonium hexafluoroantimonate, 2,2'-diphenyliodonium tetrafluoroborate, di(2,4-dichlorophenyl)iodonium hexafluorophosphate, di(4-bromophenyl)iodonium hexafluorophosphate, di(4-methoxyphenyl)iodonium hexafluorophosphate, di(3-carboxyphenyl)iodonium hexafluorophosphate, di(3-methoxycarbonylphenyl)iodonium hexafluorophosphate, di(3-methoxysulfonylphenyl)iodonium hexafluorophosphate, di(4-acetamidophenyl)iodonium hexafluorophosphate, di(2-benzothienyl)iodonium hexafluorophosphate, and diphenyliodonium hexafluoroantimonate.

Of the diaryl iodonium complex salts which are suitable for use in the compositions of the invention, include diaryliodonium hexafluorophosphate, diaryliodonium hexafluoroantimonate, (4-methylphenyl)[4-(2-methylpropyl) phenyl] iodonium hexafluoroantimonate, include (4-methylphenyl)[4-(2-methylpropyl) phenyl] iodonium tetrafluoroborate, 4-octyloxyphenyl phenyliodonium hexafluoroantimonate, 4-(2-hydroxytetradecyloxyphenyl)phenyliodonium hexafluoroantimonate, and 4-(1-methylethyl)phenyl 4-methylphenyliodonium tetrakis(pentafluorophenyl)borate.

A preferred sulfonium compound of the formula (VII) is S-(phenyl)thianthrenium hexafluorophosphate of the following formula:

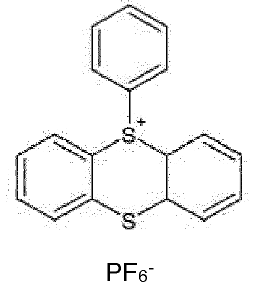

The phosphonium compound of formula (VIII) may be a tetrakis-(hydroxymethyl)-phosphonium (THP) salt or a tetrakis-(hydroxymethyl)-phosphonium hydroxide (THPOH) salt, wherein the anion A⁻ is selected from the group consisting of formate, acetate, phosphate, sulphate, fluoride, chloride, bromide and iodide.

In a salt of a compound of any one of formula (VI), (VII) and (VIII), the anion may be an anion selected from halogenides such as fluoride, chloride, bromide and iodide, sulphate, phosphate, acetate, formate, hexafluorophosphate, tetrafluoroborate, tetraphenylborate, hexafluoroantimonate, tetrakis(pentafluorophenyl)borate and trifluoromethylsulfonate.

Preferably, the metal hydride derivative is selected from the following group:
(4) a metal hydride compound of the following formula (IX):

R¹⁸R¹⁹R²⁰MH (IX)

wherein
M
Represents Ge, or Si and
R¹⁸
Represents a hydrogen atom, an organic moiety or a different moiety G;
R¹⁹ and R²⁰
which are independent from each other, represent an organic moiety.

In formula (IX), M represents Ge or Si. Preferably, M represents Ge. According to a specific embodiment, the metal hydride is a silane compoung. According to a specific embodiment, the metal hydride is a germane compound.

In formula (IX), R¹⁸ may be a hydrogen atom, an organic moiety or a different moiety G. When R¹⁸ is a hydrogen atom, then the metal complex contains two metal hydride bonds (M-H). In case R¹⁸ is a hydrogen atom, the M is Si.

When R¹⁸ is an organic moiety, R¹⁸ is preferably an aromatic, an aliphatic or an alicyclic group. An aromatic group may be a phenyl group. The phenyl group may be substituted by one or more straight chain or branched alkyl groups having 1 to 6 carbon atoms, alicyclic groups having 3 to 6 carbon atoms, halogen atoms, hydroxyl groups, or amino groups. The aliphatic group may be a straight chain or branched alkyl groups having 1 to 6 carbon atoms which may be substituted by one or more aromatic groups, alicyclic groups having 3 to 6 carbon atoms, halogen atoms, hydroxyl groups or amino groups. An alicyclic group may be a group having 3 to 6 carbon atoms which may be substituted by one or more aromatic groups, aliphatic groups, halogen atoms, hydroxyl groups or amino groups.

When R¹⁸ is a different moiety G, the metal hydride of the formula (IX) contains a metal-metal bond. In case two moieties X, R¹⁹ and R²⁰ are present, then each X, R¹⁹ and R²⁰ may be the same or different and independently has the meaning defined by the present invention.

R¹⁹ and R²⁰ which are independent from each other, represent an organic moiety. An organic group may be an aromatic, an aliphatic or an alicyclic group. An aromatic group may be a phenyl group. The phenyl group may be substituted by one or more straight chain or branched alkyl groups having 1 to 6 carbon atoms, alicyclic groups having 3 to 6 carbon atoms, halogen atoms, hydroxyl groups, or amino groups. The aliphatic group may be a straight chain or branched alkyl groups having 1 to 6 carbon atoms which may be substituted by one or more aromatic groups, alicyclic groups having 3 to 6 carbon atoms, halogen atoms, hydroxyl groups or amino groups. An alicyclic group may be a group having 3 to 6 carbon atoms which may be substituted by one or more aromatic groups, aliphatic groups, halogen atoms, hydroxyl groups or amino groups.

According to a preferred embodiment R¹⁸, R¹⁹ and R²⁰ in the metal hydride of formula (IX) are the same and represent an aliphatic, an aromatic or an alicyclic hydrocarbon group.

According to a preferred embodiment, the metal hydride of formula (IX) is a compound of the following formulae:

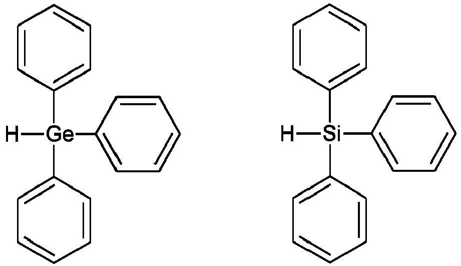

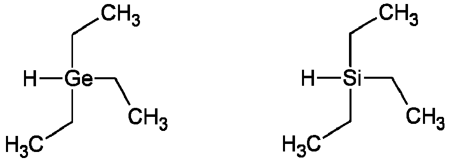

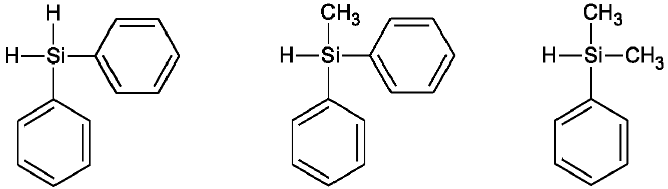

Preferably, the tertiary amine compound is selected from the following group:
(5) a tertiary amine compound of the following formula (IX):

R²¹R²²R²³N (X)

wherein
R²¹, R²² and R²³
which maybe the same or different, independently represent a substituted or unsubstituted C₁₋₄ alkyl group, a substituted or unsubstituted aryl groups.

Particularly preferred amine compounds are tertiary amines selected from the group consisting of triethanolamine, 4-N,N-dimethylaminobenzonitrile, methyl N,N-dimethylaminobenzoate, ethyl N,N-dimethylaminobenzoate, N,N-dimethylaminoethyl methacrylate and isoamyl 4-N,N-dimethylaminobenzoate, N,N-dimethylaniline, N,N-dimethyltoluidine, N,N-diethanoltoluidine, dimethylaminoanisole, 1 or 2-dimethylaminonaphthalene. In particular, the tertiary amine is selected from the group consisting of triethanolamine, methyl 4-N,N-dimethylaminobenzoate, ethyl 4-N,N-dimethylaminobenzoate, 4-N,N-dimethylaminoethyl methacrylate and isoamyl 4-N,N-dimethylaminobenzoate.

### The filler

The dental impression material of the present invention comprises a filler. The filler is a particulate filler which has preferably a mean particle size in the range of from 0.05 to 5 µm as measured, for example, by electron microscopy or by using a conventional laser diffraction particle sizing method as embodied by a MALVERN Mastersizer S or MALVERN Mastersizer 2000 apparatus. The particulate filler may be a multimodal particulate filler representing a mixture of two or more particulate fractions having different average particle sizes. The particulate reactive filler may also be a mixture of particles of different chemical composition.

Preferably, the photocurable dental impression material comprises 10 to 60 percent by weight, more preferably 20 to 50 percent by weight, based on the total weight of the dental impression material of a filler.

The specific type of filler is not particularly limited. Accordingly, any toxicologically acceptable inorganic, especially hydrophobic fillers may be employed such as silicas, aluminas, magnesias, titanias, inorganic salts, metallic oxides and glasses.

The filler may be a mixtures of different fillers such as silicone dioxides including crystalline forms, in particular particulate quartz, amorphous silicon dioxides, in particular diatomaceous earth, and silanated fumed silica.

The viscosity and thixotropicity of the uncured as well as the physical properties of the cured compositions may be controlled by varying the sizes and surface areas of the filler.

The filler may be surface treated with one or more silanating agents. Preferred silanating agents include those having at least one polymerizable double bond and at least one group that easily hydrolyses with water. Examples of such agents include 3-methacryloxypropyltrimethoxysilane, 3-methacryloxypropyldimethoxymonochlorosilane, 3-methacryloxypropyldichloromonomethoxysilane, methacryloxypropyltri-chlorosilane, 3-methacryloxypropyldichloromonomethylsilane, 3- methacryloxypropylmonochlorodimethylsilane, and mixtures thereof.

Preferred filler are fumed silica, quartz, cristobalite, calcium silicate, diatomaceous earth, zirconium silicate, montmorillonite such as bentonite, zeolite, including molecular sieves such as sodium aluminium silicate, metal oxide powder such as aluminium or zinc oxide or their mixed oxides, barium sulphate, calcium carbonate, plaster, and glass powder including a SDI glass filler.

### The reactive diluent

The curable dental impression material of the present invention may comprise a reactive diluent.

According to a specific embodiment, the curable dental impression material of the present invention comprises a reactive diluent having at least one or more cationic polymerizable groups, according to the formula (XI). 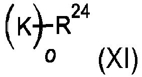 wherein
K= cationically polymerizable group
R²⁴= organic moiety
**o≥1**

Preferably, K represents a vinyl ether group, a vinyl ester group, a vinyl siloxane group, an epoxide group, an oxetane group and a furane group.

More preferably, K represents a vinyl ether group and a vinyl ester group, most preferably K represents a vinyl ether group.

Preferably, R²⁴ represents an o-valent C₁₋₃₀ hydrocarbyl groups which may contain 1-15 heteroatoms selected from O, S, Si, and which may be substituted by 1-15 substituents selected from C₁₋₄ alkyl groups, C₄₋₁₀ aryl groups, C₄₋₉ heteroaryl groups, halogen atoms, C₁₋₄ alkoxy groups, ester groups, thioether groups, silyl groups, and siloxane groups.

The hydrocarbyl group may be an alkyl group, a cycloalkyl group, a cycloalkylalkyl group, an arylalkyl group or an aryl group.

An alkyl group may be straight-chain or branched C₁₋₃₀ alkyl group, typically a C₁₋₆ alkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl.

A cycloalkyl group may be a C₃₋₂₀ cycloalkyl group, typically a C₃₋₈ cycloalkyl group. Examples of the cycloalkyl group can include those having 3 to 6 carbon atoms, for example, cyclopropyl, cyclobutyl, cyclopentyl and cyclohexyl.

A cycloalkylalkyl group may have 4 to 20 carbon atoms and may include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl.

An arylalkyl group may be a C₇₋₂₀ arylalkyl group, typically a combination of a linear or branched alkyl group having 1 to 6 carbon atoms and an aryl group having 6 to 10 carbon atoms. Specific examples of an arylalkyl group are a benzyl group or a phenylethyl group.

An aryl group can include aryl groups having 6 to 10 carbon atoms. Examples of the aryl group are phenyl and naphtyl.

The hydrocarbylcarbonyl groups of R²⁴ represent acyl groups (R²⁵-(C=O)-) in which the organic residue R²⁵ is a hydrocarbyl residue as defined above.

Preferably, the hydrocarbylcarbonyl group is an arylcarbonyl group, more preferably a benzoyl group.

Preferably, R²⁴ is selected from the group consisting of a straight chain or branched C₁₋₆ alkyl group, and a phenyl or benzoyl group which may optionally be substituted by one to three substitutents selected from halogen atoms, C₁₋₄ alkoxy groups.

Preferably, o is between 1 and 4, more preferably o is 2.

More preferably, the cationically polymerizable compound is a compound of the following formula (XII) 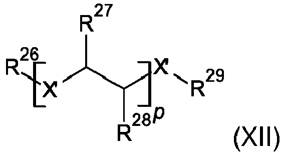 wherein
- R²⁶ and R²⁹: which may be the same or different, independently represent a hydrogen atom, a C₁₋₄ alkyl group, a vinyl group, a vinyl silyl group, an epoxide group, an oxetane group, a furane group,
- R²⁷ and R²⁸: which may be the same or different, independently represent a hydrogen atom, a C₁₋₄ alkyl group, or a vinyl ether group, a vinyl ester group, a vinyl siloxane group, an epoxide group, an oxetane group, a furane group,
- X': represents an oxygen, a sulfur or a carbon atom,
- p: represents an integer of from 1 to 10, provided that at least one cationic polymerizable group is present in the compound of formula (XII).

In a preferable embodiment, X' represents an oxygen atom, R²⁷ represents a hydrogen atom, or a methyl group, R²⁸ represents a hydrogen atom, R²⁶ and R²⁹ represent vinyl groups, more preferable X' represents an oxygen atom, R²⁷ and R²⁸ represent a hydrogen atom, R²⁶ and R²⁸ represent vinyl groups.

A particular suitable reactive diluent is ethylene glycol vinyl ether.

The reactive diluent may be contained in the curable dental impression material of the present invention in an amount of up to 15 percent by weight based on the total weight of the composition.

### Optional polymerizable (meth)acrylates or (meth)acrylamides

The dental impression material of the present invention may further comprise up to 20 percent by weight based on the total weight of the composition of polymerizable (meth)acrylates or (meth)acrylamides.

The (meth)acrylate compounds may be selected from methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, bisphenol A glycerolate dimethacrylat ("bis-GMA", CAS-No. 1565-94-2), 4,4,6,16 (or 4,6,6,16)-tetramethyl-10,15-dioxo-11,14-dioxa-2,9-diazaheptadec-16-enoicacid 2-[(2-methyl-1-oxo-2-propen-1-yl)oxy]ethyl ester (CAS no. 72869-86-4)_(UDMA), glycerol mono-and di- acrylate such as 1,3-glycerol dimethacrylate (GDM), glycerol mono-and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane, and 2,2'-bis[3(4-phenoxy)-2-hydroxypropane-1-acrylate]propane.

Preferred (meth)acrylamides may be selected from the following compounds.

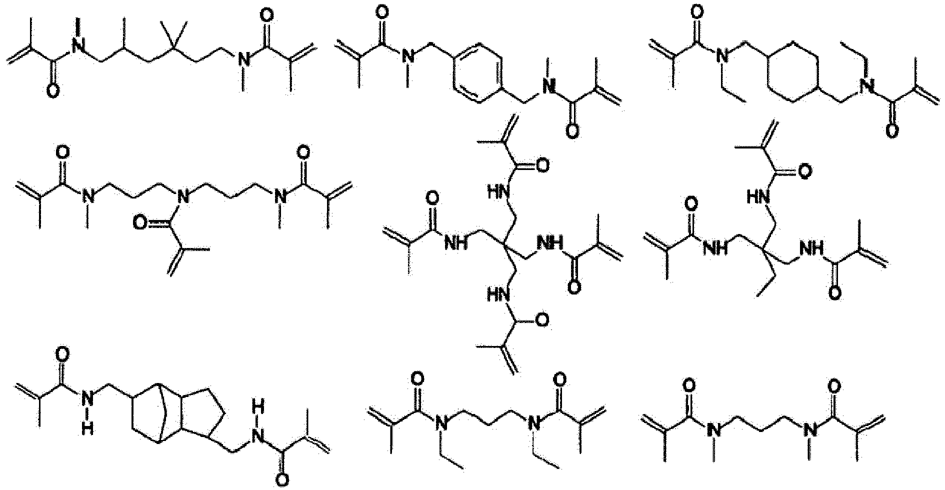

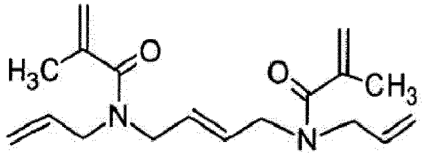

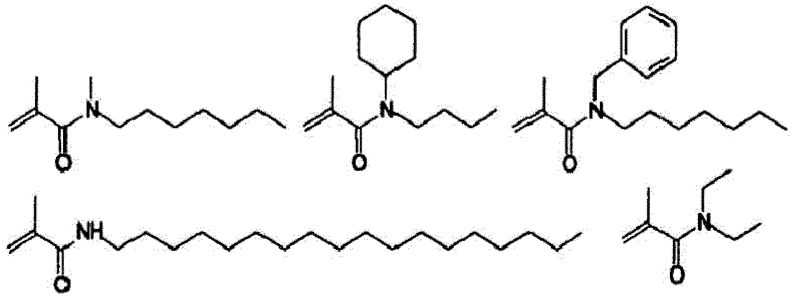

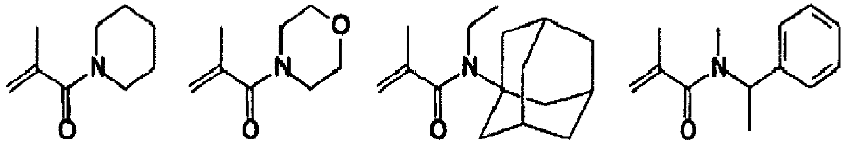

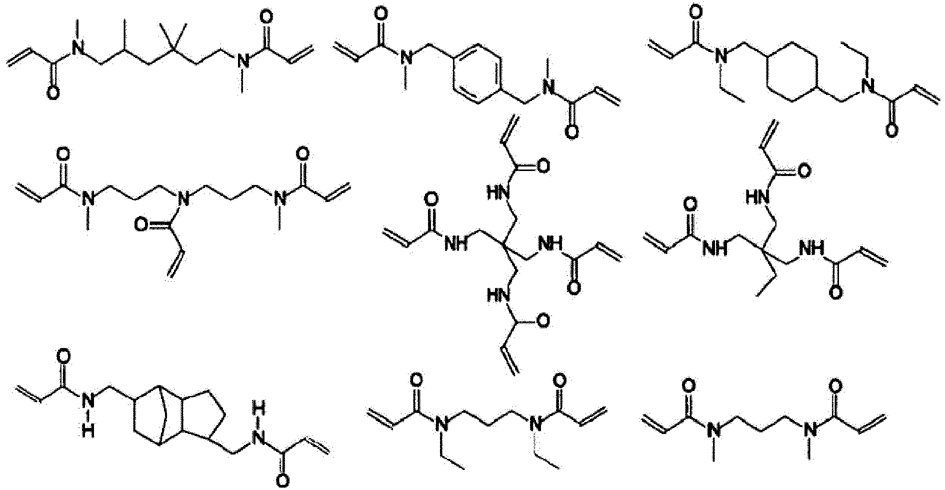

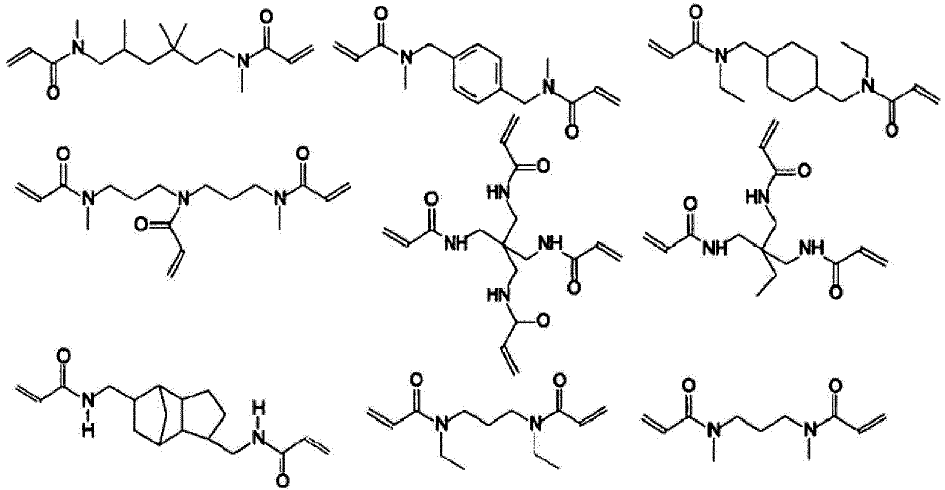

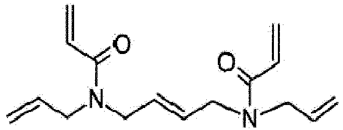

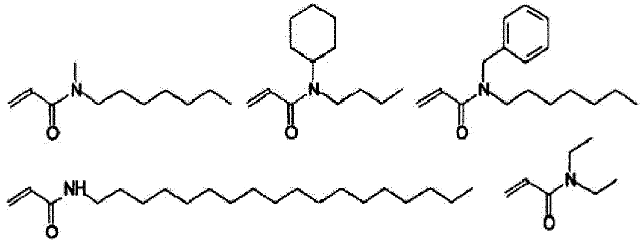

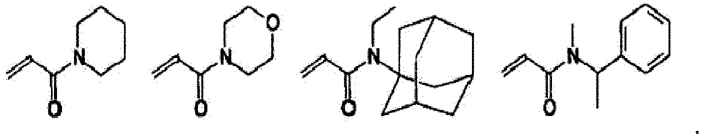

Most preferred are the bis-(meth)acrylamides:
N,N'-diallyl-1,4- bisacrylamido-(2E)-but-2-en (BAABE) having the structural formula 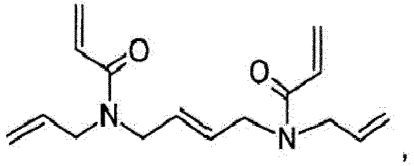 and
N,N'-diethyl-1,3-bisacrylamido-propan (BADEP) having the structural formula

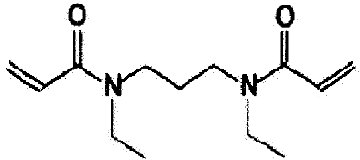

### Further components

Optionally, the dental impression material of the present invention may further comprise stabilizer(s), and/or pigments.

The term "stabilizer" as used herein means any compound capable of preventing polymerizable compounds contained in the dental impression material from spontaneous polymerization during storage. However, the stabilizer does not disturb or prevent intended polymerisation curing of the dental impression material during application.

For example, the stabilizer may be a conventional stabilizer selected from the group consisting of hydroquinone, hydroquinone monomethylether, tert-butylhydroquinone, tert-butylhydroxyanisol, propyl gallate and 2,6-di-tert-butyl-p-cresol. From these conventional stabilizers, 2,6-di-tert-butyl-p-cresol is preferred.

The dental impression material according to the invention may contain the stabilizer in an amount of 0.001 to 1 percent by weight, preferably 0.005 to 0.8 percent by weight based on the total weight of the composition. When the amount of the stabilizer is below the above indicated lower limit of 0.001, then storage stability of the dental impression material might be insufficient, since the amount of stabilizer is too small to provide a stabilizing effect. However, when the amount of stabilizer is above the maximum threshold of 1 percent by weight, then the applicability of the dental impression material might be negatively affected, since higher amounts of stabilizer may disturb or even substantially prevent intended polymerisation curing of the dental impression material during application.

The present invention provides a method for preparing a dental impression.

The method comprises providing a curable dental impression material according to the present invention.

Moreover, the method further comprises taking an impression of a dental structure.

Finally, the method of the present invention comprises curing the dental impression material with light having a wavelength in the range of 200 to 800 nm.

The present invention also provides the use of the photocurable dental impression material according to the present invention for the preparation of a dental impression.

The present invention will now be further illustrated based on the following examples.

### Examples

The compounds investigated to develop photoinitiating systems are given in Scheme 1. The monomers used for cationic polymerization are given in Scheme 2. All these chemical compounds are commercially available and were used with the highest purity available.

### 1) Initiators and additives

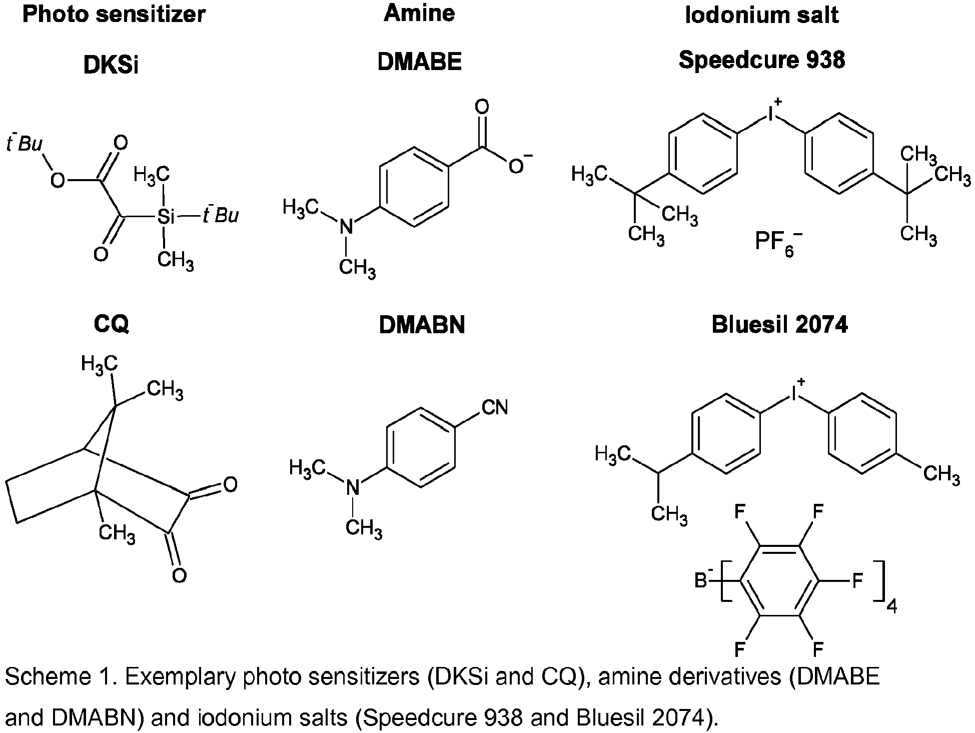

### 2) Investigated epoxy-silicone monomers

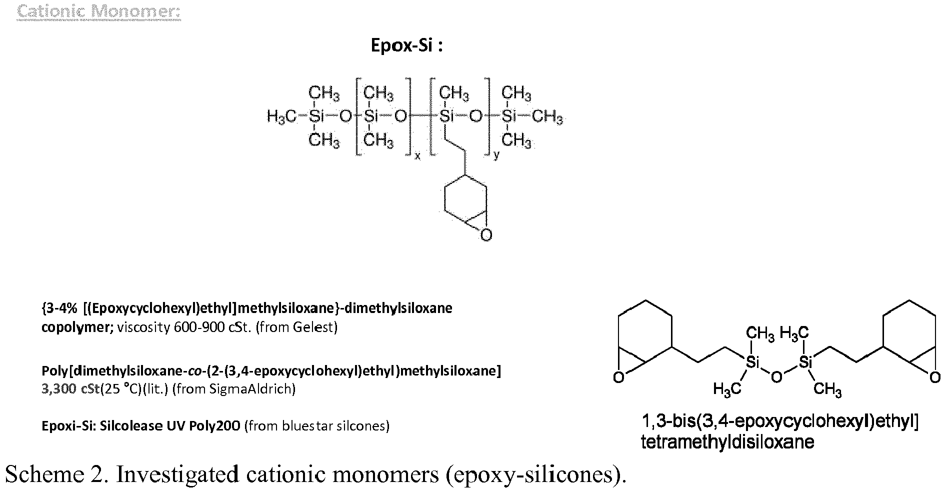

### Example 1. Cationic polymerization of 1,3-bis[2-(3,4-epoxycyclohexyl)ethyl]tetramethyldisiloxane

### Example 1a.

2 g of 1,3-bis[2-(3,4-epoxycyclohexyl)ethyl]tetramethyldisiloxane were mixed with 20 mg of diphenyliodonium hexafluorophosphate, 20 mg of camphor quinone and 20 mg of 4-(dimethylamino)benzoate. As the DPI was not dissolved, 0.2 g of ethylene glycol vinyl ether were added. The homogeneous mixture was cured after exposure of 20 s to the Smart Lite Focus (Dentsply Sirona).

### Example 1b.

2 g of 1,3-bis[2-(3,4-epoxycyclohexyl)ethyl]tetramethyldisiloxane were mixed with 20 mg of diphenyliodonium hexafluorophosphate, 20 mg of camphor quinone and 20 mg of triphenylgermanium hydride. As the DPI was not dissolved, 0.2 g of ethylene glycol vinyl ether were added. The homogeneous mixture was cured after exposure of 20 s to the Smart Lite Focus (Dentsply Sirona).

### Example 1c.

1,3-bis[2-(3,4-epoxycyclohexyl)ethyl]tetramethyldisiloxane was mixed with 1 wt % of the specific DPI, 1 wt % of camphor quinone and optionally with 1 wt % of 4-(dimethylamino)benzoate or triphenylgermanium hydride. The results of the Photo DSC measurements are presented in table 1.

**Table 1. Curing behavior and Photo-DSC measurement**

| Example | Photo-Initiator (1 wt%) | DPI (1 wt%) | Curing (Smart Lite Focus) | **Δ** H [J/g] | Peak [min] |
|---|---|---|---|---|---|
| 1A | CQ | Speedcure 938 | Gelation after 60 s | -3.5 | 0.146 |
| 1B | CQ | PI 2074 | Solid after 20 s | -34.3 | 10.4 |
| 2A | CQ/DMABE | Speedcure 938 | Solid after 20 s | -22.8 | 4.0 |
| 2B | CQ/DMABE | PI 2074 | Solid after 20 s | - 103.9 | 1.4 |
| 3A | CQ/TPGH | Speedcure 938 | Solid after 20 s | - 10.8 | 0.6 |
| 3B | CQ/TPGH | PI 2074 | Solid after 20 s | -310.4 | 0.7 |

| | | | | | |
|---|---|---|---|---|---|
| CQ: camphor quinone DMABE: 4-(dimethylamino)benzoate TPGH: triphenylgermanium hydride | | | | | |

### Example 1d.

1,3-bis[2-(3,4-epoxycyclohexyl)ethyl]tetramethyldisiloxane was mixed with 1 wt % of the specific DPI, 1 wt % of tert-Butyl(tert-butyldimethyl-silyl)glyoxylate and optionally with 1 wt % of 4-(dimethylamino)benzoate or triphenylgermanium hydride. The results of the Photo DSC measurements are presented in table 2.

**Table 2. Curing behavior and Photo-DSC measurement at 120 mW/cm²**

| Ex. | Photo-Initiator (1 wt%) | DPI (1 wt%) | Curing (Smart Lite Focus (SL) or Licu Lite (LL)) | Expos ure time [min] | **Δ H** [J/g] | Peak [min] |
|---|---|---|---|---|---|---|
| B | DKSi | PI 2074 | Solid after 6 min LL | 30.1 | - 71.0 | 15.8 |
| C | DKSi / DMABE | Speedcure 938 | Gelation after 6 min LL | 8.1 | - 10.7 | 0.15 |
| D | DKSi / DMABE | PI 2074 | Gelation after 20 s SL | 8.1 | - 8.4 | 4.6 |
| E | DKSi / TPGH | Speedcure 938 | Gelation after 6 min LL | 8.1 | - 1.8 | 0.1 |
| F | DKSi / TPGH | PI 2074 | Solid after 20 s SL | 20.1 | - 322.1 | 3.4 |

| | | | | | | |
|---|---|---|---|---|---|---|
| DMABE: 4-(dimethylamino)benzoate TPGH: triphenylgermanium hydride DKSi: tert-Butyl(tert-butyldimethyl-silyl)glyoxylate | | | | | | |

### Example 2. Cationic polyermization profiles of epoxy-siloxanes

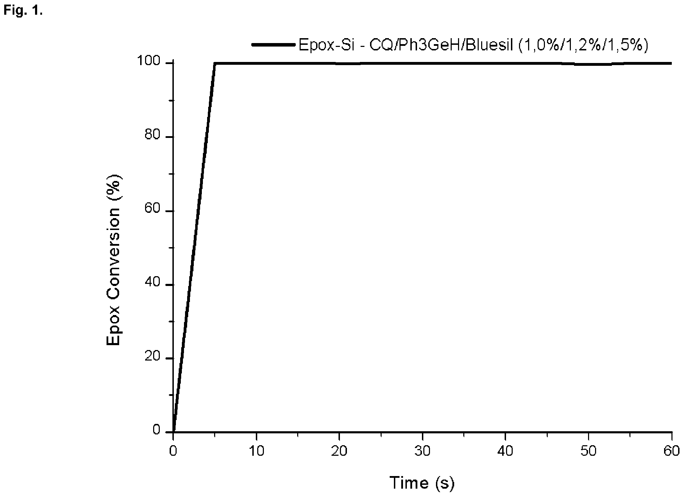

Different initiating systems based on camphorquinone/amine/iodonium salt or CQ/triphenylgermanium hydride (Ph₃GeH)/iodonium salt or DKSi/iodonium salt (and optionally amine or triphenylgermanium hydride (Ph₃GeH)) were used to initiate the polymerization of epoxy-silicones (Scheme 2). Remarkably, excellent polymerization profiles were obtained for DKSi or CQ based initiating systems upon blue light and under air showing their high performance (Figure 1). Tack free polymers can be obtained. The presence of iodonium salt is clearly required i.e. excellent final conversions are reached under air and for samples of thickness 1.4 mm for a Dental LED (λₘₐₓ ∼ 480 nm) only in presence of iodonium salt. Without CQ or DKSi, no polymerization occurs showing the role of the photoinitiator absorbing blue light.

### Example 3. Depth of cure upon blue-light for epoxy-siloxane photopolymerization

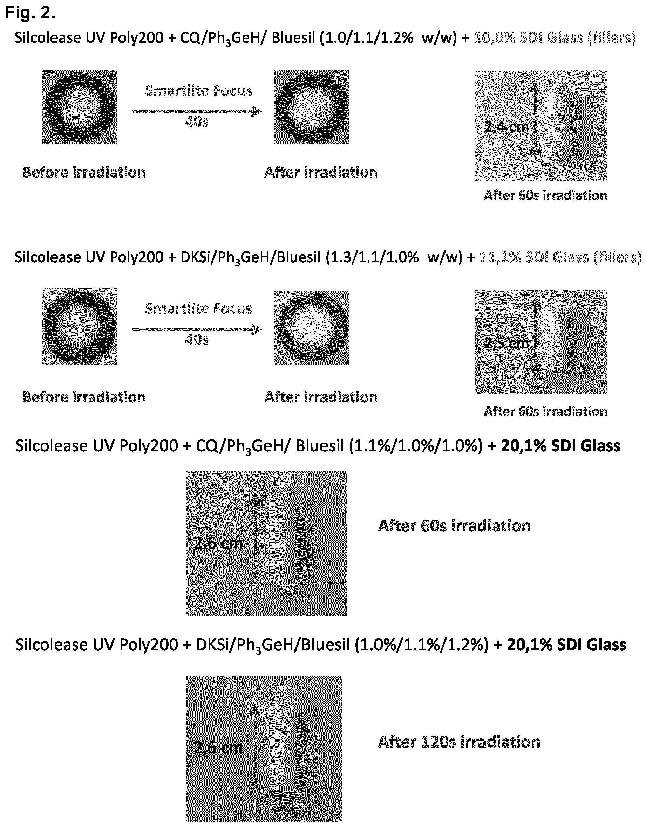

The Depth of cure (DoC) in presence of fillers (10 or 20% w/w) is found excellent (Figure 2); excellent bleaching properties for the surface under irradiation are also found (Figure 2).

## Claims

1. A curable dental impression material comprising:
(a) one or more siloxane compounds having two or more epoxide groups;
(b) a photo initiator for initiating cationic polymerization of epoxide groups of the one or more siloxane compounds; and
(c) a filler.

2. The curable dental impression material according to claim 1, which further comprises
(d) a reactive diluent having two or more vinyl ether groups.

3. The curable dental impression material according to claim 1 or 2, wherein the siloxane compound is a compound of the following formula (I): 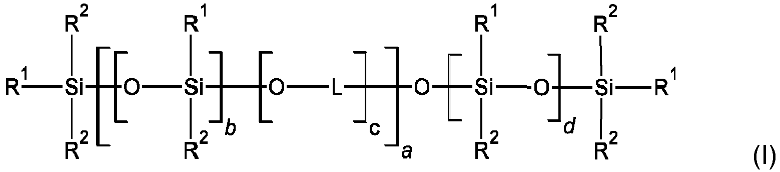 wherein
R¹ which may be the same or different, independently represents an epoxide-group containing organic residue, a C₁₋₄ alkyl group, a group of the formula [**-OSiR'**₂]ₙ**R'**, or two groups **R¹** or a group R¹ and R² form together a group [**-OSiR'**₂]ₙ-, wherein the **R'**, which may be the same or different, independently represent a C₁₋₄ alkyl group or an epoxide-group containing organic residue and n is an integer of from 1 to 20;
R² which may be the same or different, independently represents a C₁₋₄ alkyl group or a group of the formula [**-OSiR"₂**]ₘ**R"**, or two groups R² or a group R¹ and R² form together a group [**-OSiR"**₂]ₘ-, wherein the **R", which may be the same or different, independently represent a C₁₋₄** alkyl group and m is an integer of from 1 to 20;
L represents a divalent C₁₋₂₀ organic linker group;
a represents 0 or an integer of from 1 to 20;
b represents 0 or an integer of from 1 to 20;
c represents 0 or an integer of from 1 to 20;
d represents 0 or an integer of from 1 to 20;
provided that at least two epoxy groups are present in the compound of formula (I).

4. The curable dental impression material according to claim 3, wherein the compound of formula (I) has an average molecular weight of 200 to 10000 Da.

5. The curable dental impression material according to any one of claims 3 or 4, wherein the dynamic viscosity (25 °C) of the compound of formula (I) is in the **range of from 1 to 100 Pa·s.**

6. The curable dental impression material according to any one of claims 1 to 5, wherein the epoxide-group containing organic residues are cycloaliphatic epoxide groups of the following formula (II): 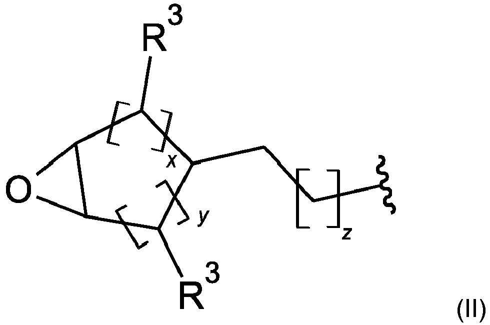 wherein
R³ which may be the same or different, independently represent a hydrogen atom or a C₁₋₄ alkyl group;
x is 0 or an integer of from 1 to 4;
y is 0 or an integer of from 1 to 4;
z is an integer of from 1 to 3; and
(x+y) is 2 to 8.

7. The curable dental impression material according to any one of the preceding claims wherein the photo initiator compound is selected from diaryliodonium salts, triarylsulfonium salts, tetraarylphosphonium salts, or pyridinium salts, and camphor quinone, or camphor quinone/germanium hydride derivative, or camphor quinone/amine derivative, or DKSi, or DKSi/ germanium hydride derivative, or DKSi/amine derivative.

8. The curable dental impression material according to any one of the preceding claims, wherein the photo initiator comprises:
(1) an diaryliodonium compound of the following formula (III):
R⁹-I⁺-R¹⁰ A⁻ (III)
wherein
R⁹ and R¹⁰
which are independent from each other, represent an optionally substituted aryl group, and
A⁻ is an anion; and/or
(2) a triarylsulfonium compound of the following formula (IV):
R¹¹R¹²R¹³S⁺ A⁻ (IV)
wherein
R¹¹, R¹² and R¹³
which are independent from each other, represent an optionally substituted aryl group or wherein any two of R¹¹, R¹² and R¹³ form a cyclic aromatic structure together with the sulfur atom to which they are bound, and
A⁻ is an anion; and/or
(3) a tetraarylphosphonium compound of the following formula (V):
R¹⁴R¹⁵R¹⁶R¹⁷P⁺ A⁻ (V)
wherein
R¹⁴ R¹⁵, R¹⁶ and R¹⁷
which are independent from each other, represent an optionally substituted aryl group or wherein any two of R¹⁴, R¹⁵, R¹⁶ and R¹⁷ form a cyclic aromatic structure together with the phosphor atom to which they are bound, and
A⁻ is an anion; and/or
(4) a germanium hydride compound of the following formula (VI):
R¹⁸R¹⁹R²⁰MH (VI)
wherein
M
represents Ge
R¹⁸, R¹⁹ and R²⁰
which are independent from each other, represent an aryl group; and/or
(5) a tertiary amine compound of the following formulat (VII):
R²¹R²²R²³N (VII)
wherein
R²¹, R²² and R²³
which maybe the same or different, independently represent a substituted or unsubstituted C₁₋₄ alkyl group, a substituted or unsubstituted aryl groups.

9. The curable dental impression material according to any one of the preceding claims, wherein the photo initiator comprises a sensitizer having an absorption maximum in the range of from 400 to 800 nm, preferably selected from camphor quinone, and tert.-butyl(tert.-butyldimethylsilyl)glyoxylate.

10. The curable dental impression material according to any one of the preceding claims, which comprises 40 to 90 percent by weight based on the total weight of the dental impression material of siloxane compounds having two or more epoxide groups.

11. The curable dental impression material according to any one of the preceding claims, which comprises 10 to 60 percent by weight based on the total weight of the dental impression material of a filler.

12. The curable dental impression material according to any one of the preceding claims, wherein the filler has a mean particle size in the range of from 0.05 to 5 µm.

13. A method for preparing a dental impression, which method comprises
(a) providing a curable dental impression material as defined by any one of claims 1 to 12;
(b) taking an impression of a dental structure;
(c) curing the dental impression material with light having a wavelength in the range of 200 to 800 nm.

14. Use of the curable dental impression material as defined by any one of claims 1 to 12, for the preparation of a dental impression.
